(19) Europäisches Patentamt
European Patent Office
Office européen des brevets

(11) **EP 2 965 683 A1**

(12) **EUROPEAN PATENT APPLICATION**
published in accordance with Art. 153(4) EPC

(43) Date of publication:
**13.01.2016 Bulletin 2016/02**

(51) Int Cl.:
***A61B 1/00*** (2006.01)  ***A61B 1/04*** (2006.01)
***A61B 1/06*** (2006.01)

(21) Application number: **14759490.7**

(22) Date of filing: **03.03.2014**

(86) International application number:
**PCT/JP2014/055235**

(87) International publication number:
**WO 2014/136706 (12.09.2014 Gazette 2014/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **06.03.2013 JP 2013044282**

(71) Applicant: **Olympus Corporation
Shibuya-ku
Tokyo 151-0072 (JP)**

(72) Inventors:
• **NISHIO, Masahiro
Tokyo 151-0072 (JP)**
• **ITO, Takeshi
Tokyo 151-0072 (JP)**
• **YAMAMOTO, Eiji
Tokyo 151-0072 (JP)**

(74) Representative: **Winter, Brandl, Fürniss, Hübner,
Röss, Kaiser, Polte - Partnerschaft mbB
Patent- und Rechtsanwaltskanzlei
Alois-Steinecker-Strasse 22
85354 Freising (DE)**

(54) **SUBJECT OBSERVATION SYSTEM AND METHOD**

(57) A subject observation system includes primary light sources (5, 6), a wavelength converting section (12), an image acquiring section (3), a mode input section (13), and a light source section (2). The wavelength converting section (12) converts wavelengths of plural primary light of different wavelengths emitted from the primary light sources, respectively. The light source section (2) successively lights the primary light sources to successively irradiate the observation target with the plural illuminating light from an aperture of a same position, when a special light observation mode to emphasize and display a specific observation target in the observation target is input into the mode input section. The image acquiring section (3) acquires the images every time the observation target is irradiated with respective illuminating light, and generates a special light observation image of the observation target based on the images.

F I G. 1

EP 2 965 683 A1

## Description

Technical Field

The present invention relates to a subject observation system which performs observations such as a normal observation by white light and an observation by light of a wavelength different from a wavelength in the normal observation, e.g., special light to observe a specific subject, and to a subject observation method.

Background Art

For example, a subject observation system such as an endoscope includes a light emitting device to irradiate a subject with white light or the like. As the light emitting device, at present, a device has been developed in which a wavelength converting member is disposed at an optical fiber distal end and a wavelength of light output from a small solid light source is converted by the wavelength converting member, whereby the light is changed into light having a desirable irradiation pattern or color. Jpn. Pat. Appln. KOKAI Publication No. 2005-205195 (PTL 1) discloses a light emitting device that is capable of emitting various colors by a combination of an excitation light source with the wavelength converting member disposed at the optical fiber distal end, and an endoscope device in which the light emitting device is used.

Citation List

Patent Literature

PTL 1: Jpn. Pat. Appln. KOKAI Publication No. 2005-205195

Summary of Invention

Technical Problem

In PTL 1, wavelength converting members disposed at a distal end are excited by an excitation light source, white light generated by this excitation, i.e., the white light having spectral components in a broad range of a visible light region, is emitted as the light of a light source for an endoscope, and a living body is irradiated with this white light to realize a normal light observation in the living body. In PTL 1, when light having a color different from the white light, i.e., light having a different wavelength, is emitted, the wavelength converting members having different light emission characteristics are excited by the excitation light source to realize the observation.

In PTL 1, when observations such as the normal light observation by the white light and the observation by the color different from that of the white light are conducted, it is necessary to dispose at the distal end the wavelength converting members having the different light emission characteristics. In PTL 1, light guide members that guide the excitation light from the excitation light source to the wavelength converting members are required, and excitation light sources are also required in certain cases, thus making the miniaturization of a light emitting device or the like difficult.

An object of the present invention is to provide a subject observation system which is usable in observations such as a normal light observation and a special light observation, and which is capable of obtaining an image of a desired wavelength band and realizing miniaturization, and to provide a subject observation method.

Solution to Problem

A subject observation system according to an aspect of the invention includes primary light sources, a wavelength converting section which converts wavelengths of plural primary light of different wavelengths emitted from the primary light sources, respectively, an image acquiring section which performs color separation by color regions to acquire an image of an irradiated region of illuminating light in an observation target, when the observation target is irradiated with the illuminating light including the light whose wavelength has been converted by the wavelength converting section, a mode input section which is able to input observation modes, and a light source section which successively lights the primary light sources to successively irradiate the observation target with the plural illuminating light from an aperture of a same position, when a special light observation mode to emphasize and display a specific observation target in the observation target is input into the mode input section, wherein the image acquiring section acquires the images every time the observation target is irradiated with respective illuminating light, and generates a special light observation image of the observation target based on the images.

A subject observation method according to an aspect of the invention includes converting wavelengths of plural primary light of different wavelengths which are emitted from primary light sources, respectively, irradiating an observation target with plural illuminating light including light whose wavelength has been converted, performing color separation by color regions to acquire images of irradiated regions with the plural illuminating light in the observation target, successively lighting the primary light sources to successively irradiate the observation target with the plural illuminating light from an aperture of a same position, in a special light observation mode to emphasize and display a specific observation target in the observation target, acquiring the images every time the observation target is irradiated with respective illuminating light, and generating a special light observation image of the observation target based on the images.

Advantageous Effects of Invention

According to the present invention, it is possible to provide a subject observation system which is usable in observations such as a normal light observation and a special light observation, and which is capable of obtaining an image of a desired wavelength band and realizing miniaturization, and to provide a subject observation method.

Brief Description of Drawings

FIG. 1 is a configuration diagram showing a first embodiment of a subject observation system according to the present invention;

FIG. 2 is a schematic configuration diagram showing a wavelength conversion unit in the device;

FIG. 3 is a diagram showing excitation/fluorescent spectral characteristics of an oxide fluorescent material forming a wavelength converting member in the device;

FIG. 4 is a diagram showing sensitivity wavelength characteristics of a CCD imager for use in an imaging means in the device;

FIG. 5 is a diagram showing spectral characteristics of first illuminating light emitted from the wavelength conversion unit in the device;

FIG. 6 is a diagram showing spectral characteristics of second illuminating light emitted from the wavelength conversion unit in the device;

FIG. 7 is a diagram showing an absorption coefficient that becomes an index of an absorption intensity of hemoglobin flowing in a blood vessel K of a biological tissue in the device;

FIG. 8 is a diagram showing a part corresponding to a second narrowband spectrum element in which light receiving sensitivity characteristics of a G pixel of the imaging means in the device overlap with a wavelength spectrum of yellow fluorescence;

FIG. 9 is a configuration diagram showing a wavelength conversion unit in a second embodiment of the subject observation system according to the present invention;

FIG. 10 is a diagram showing excitation/fluorescence characteristics of a wavelength converting member (green) in the device;

FIG. 11 is a diagram showing excitation/fluorescence characteristics of a wavelength converting member (red) in the device;

FIG. 12 is a configuration diagram showing a wavelength conversion unit in a third embodiment of the subject observation system according to the present invention.

Description of Embodiments

[First Embodiment]

Hereinafter, a first embodiment of the present invention will be described with reference to the drawings.

A relation between a color region and a wavelength region in the present embodiment is defined as follows. A blue (B) region is defined as a wavelength region of 380 nm to 500 nm. A green (G) region is defined as a wavelength region of 500 nm to 600 nm. A red (R) region is defined as a wavelength region of 600 nm to 720 nm. Wavelengths at which sensitivities of an imaging section 30 to a blue pixel, a green pixel, and a red pixel are maximized are included in the blue region, the green region, and the red region, respectively.

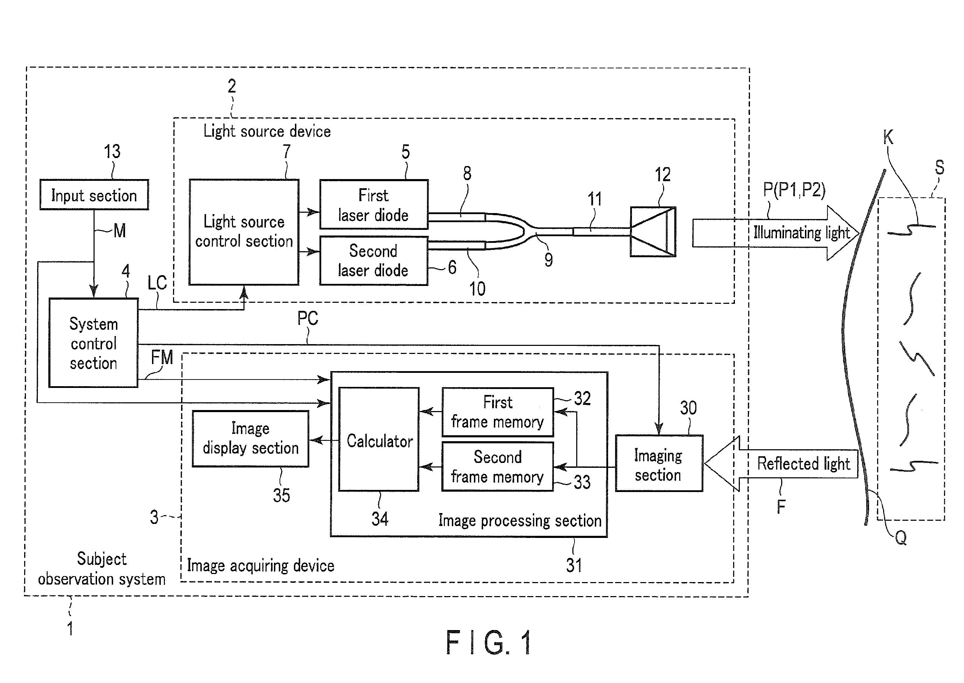

FIG. 1 shows a configuration diagram of a subject observation device 1 including a light source device. The subject observation device 1 observes an observation target in a subject Q such as a living body, e.g., hemoglobin flowing in a blood vessel K. The subject Q is, for example, a human body or the like, and a biological tissue including the blood vessels (including capillary blood vessels, blood vessels with thick walls, etc.) K.

The subject observation device 1 has observation modes such as a normal light observation mode and a special light observation mode. In the normal light observation mode, the subject Q is irradiated with white light and observed. In the special light observation mode, a specific observation target among the observation targets is emphasized and displayed. Specifically, in the special light observation mode, for example, hemoglobin flowing in the blood vessel K is emphasized and displayed as the observation target in the subject Q.

The subject observation device 1 includes a light source device 2 that irradiates the subject Q with first or second illuminating light P1 or P2 as illuminating light P, an image acquiring device 3 that acquires image information of the subject Q, and a system control section 4 that controls the light source device 2 and the image acquiring device 3.

The light source device 2 includes excitation light sources as primary light sources, and a wavelength converting section. The excitation light sources output primary light of different wavelengths. The wavelength converting section receives the primary light output from the excitation light sources to convert wavelengths, and emits the illuminating light P including the light whose wavelength has been converted, to the observation target.

Specifically, the light source device 2 includes the excitation light sources (the primary light sources) which emit the excitation light having different spectra, respectively. The excitation light sources include, for example, a first laser diode 5 that is a semiconductor laser as a first excitation light source, and a second laser diode 6 that is a semiconductor laser as a second excitation light source.

The first laser diode 5 emits, as first primary light (first excitation light), laser light of a blue-violet color of a narrowband in which a center wavelength (first spectrum) light emission peak is 415 nm ($\lambda$1) and a half-value width is several nm or less.

The second laser diode 6 emits, as second primary light (second excitation light), laser light of a blue color of a narrowband in which a center wavelength (second spectrum) light emission peak is 445 nm ($\lambda$2) and a half-value width is several nm or less.

The respective laser light (the first and second excitation light) emitted from the first and second laser diodes 5 and 6 is present in the blue region. In the wavelengths $\lambda$1 and $\lambda$2 of the respective laser light emitted from the respective laser diodes 5 and 6, examples of representative values include center wavelengths of 415nm ($\lambda$1) and 445 nm ($\lambda$2) which become light emission peaks, respectively, but the present invention is not limited to these center wavelengths, and there may be variances/individual differences in a range in which an operation and an effect of the present embodiment can be maintained.

The first and second laser diodes 5 and 6 are connected to a light source control section 7, and driven and controlled by the light source control section 7. Details of the driving control of the first and second laser diodes 5 and 6 by the light source control section 7 will be described later.

The first laser diode 5 is optically connected to one entrance end of an optical coupler 9 via a first optical fiber 8. The first optical fiber 8 guides, to the optical coupler 9, the laser light of the blue-violet color emitted from the first laser diode 5.

The second laser diode 6 is optically connected to the other entrance end of the optical coupler 9 via a second optical fiber 10. The second optical fiber 10 guides, to the optical coupler 9, the laser light of the blue color emitted from the second laser diode 6.

An emission end of the optical coupler 9 is optically connected to a wavelength conversion unit 12 via a third optical fiber 11. The optical coupler 9 emits the blue-violet laser light guided by the first optical fiber 8 as the first primary light (the excitation light), emits blue laser light guided by the second optical fiber 10 as the second primary light, or emits primary light (mixed excitation light) in which the first primary light, which is the blue-violet laser light guided by the first optical fiber 8, is mixed with the second primary light that is the blue laser light guided by the second optical fiber 10.

The third optical fiber 11 guides, to the wavelength conversion unit 12, the primary light mixed and emitted by the optical coupler 9, i.e., the blue-violet laser light, the blue laser light, or the mixed excitation light. The third optical fiber 11 irradiates the subject Q with the blue-violet laser light, the blue laser light, or the mixed excitation light from an aperture of the third optical fiber 11, i.e., the aperture of the same position.

Each of the first, second, and third optical fibers 8, 10, and 11 is a multimode optical fiber having, e.g., a core diameter of 50 $\mu$m and a numerical aperture FNA = 0.2.

Coupling lenses are interposed between the first and second laser diodes 5 and 6 and the optical fibers 8 and 10, respectively. The coupling lenses converge the blue-violet laser light and the blue laser light emitted from the first and second laser diodes 5 and 6, respectively, to couple the lights to the respective optical fibers 8 and 10.

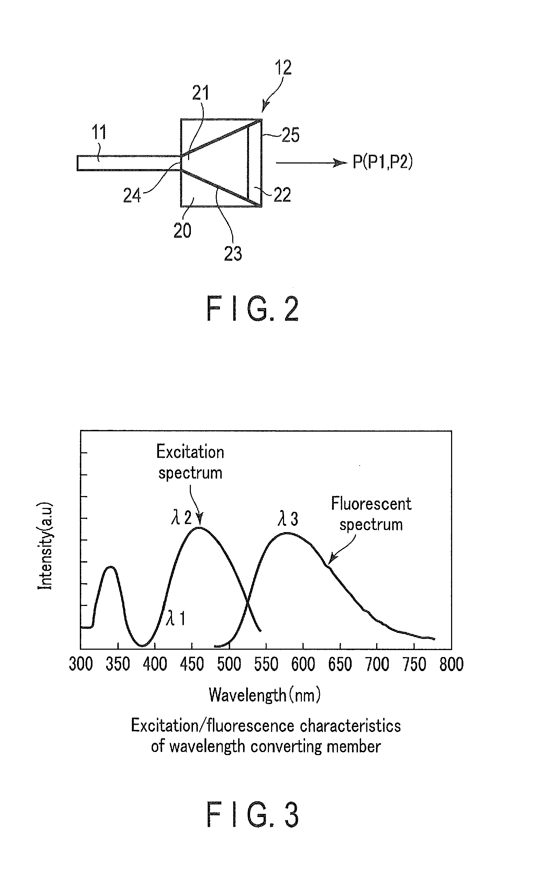

FIG. 2 shows a schematic configuration diagram of the wavelength conversion unit 12. The wavelength conversion unit 12 receives the primary light emitted from the third optical fiber 11, i.e., the blue-violet laser light, the blue laser light, or the mixed excitation light, and emits the first or second illuminating light P1 or P2 as the illuminating light P in accordance with this excitation light.

Specifically, the wavelength conversion unit 12 has a low excitation intensity to the blue-violet laser light ($\lambda$1) as the primary light emitted from the third optical fiber 11, dose not convert the wavelength and does not emit any fluorescence. As a result, the wavelength conversion unit 12 transmits the blue-violet laser light ($\lambda$1), and emits the blue-violet laser light ($\lambda$1) as the illuminating light (observation light) P, i.e., as the first illuminating light P1 here. The first illuminating light P1 includes at least a first narrowband spectrum element constituted of the first primary light emitted from the first laser diode 5 as the first primary light source in the first and second laser diodes 5 and 6. In the first illuminating light P1, an absorption intensity of a substance of the subject Q is low at a wavelength that is relatively hard to be absorbed, in a first color region in the color regions including the first narrowband spectrum element.

The wavelength conversion unit 12 converts the wavelength of the blue laser light ($\lambda$2) as the primary light emitted from the third optical fiber 11, i.e., converts the wavelength to that of yellow fluorescence ($\lambda$3) here, and emits the fluorescence ($\lambda$3). In addition, the blue laser light ($\lambda$2), whose wavelength is not converted by the wavelength conversion unit 12 but which is transmitted through the wavelength conversion unit 12, is present.

Therefore, the wavelength conversion unit 12 mixes the fluorescence ($\lambda$3) whose wavelength has been converted with a part of the blue laser light ($\lambda$2) whose wavelength is not converted by the wavelength conversion unit 12, but which is transmitted through the wavelength conversion unit, and emits the mixed light as the illuminating light (the observation light) P, i.e., as the second illuminating light P2 here. The second illuminating light P2 includes the light in which the second primary light emitted from the second laser diode 6 as the second primary light source in the primary light sources is mixed with first fluorescence generated by a first fluorescent material (a wavelength converting member 22) excited by the second primary light.

A structure of the wavelength conversion unit 12 includes a holder 20, a light transmitting member 21, the wavelength converting member 22, a reflecting member 23, an entrance portion 24, and an emitting portion 25.

The holder 20 holds the light transmitting member 21 and the wavelength converting member 22. In the holder 20, the light transmitting member 21 is disposed on a connection side of the third optical fiber 11, i.e., an entrance side of the primary light. On an emission side of the illuminating light P of the holder 20, the wavelength converting member 22 is disposed. On a light transmitting member 21 side of the holder 20, the entrance portion 24 to allow entrance of the primary light is formed. On a wavelength converting member 22 side of the holder 20, the emitting portion 25 is formed to emit the illuminating light P that is the first or second illuminating light P1 or P2.

An inner portion of the holder 20 is formed into a conically tapered shape dented in the form of, e.g., a concave portion. On an inner peripheral surface of the tapered shape, for example, the film-like reflecting member 23 is formed. The reflecting member 23 performs regular reflection or diffusion reflection of the laser light and the fluorescence.

The light transmitting member 21 transmits the primary light (the blue-violet laser light, the blue laser light, and the mixed light) emitted from the emission end of the third optical fiber 11 and the fluorescence whose wavelength has been converted by the wavelength converting member 22. The light transmitting member 21 is formed of a member of glass or silicone resin having a high transmittance.

The wavelength converting member 22 absorbs the blue laser light (the center wavelength of 445 nm: the second excitation light: $\lambda 2$) of the primary light emitted from the second laser diode 6 to emit the yellow fluorescence ($\lambda 3$). In the wavelength converting member 22, for example, an oxide fluorescent material (YAG, TAG) having a Ce (cerium) activated garnet crystal structure is used. The oxide fluorescent material (YAG, TAG) is a material that is capable of absorbing the blue laser light of a wavelength region of 430 nm to 470 nm to emit the yellow fluorescence. Thus, the oxide fluorescent material (YAG, TAG) is usable in combination with the blue laser light having a peak in the wavelength region of 430 nm to 470 nm.

The wavelength converting member 22 has a low excitation intensity to the laser light of the blue-violet color (the center wavelength of 415 nm: the first excitation light: $\lambda 1$) emitted from the first laser diode 5, and is not excited.

FIG. 3 shows excitation/fluorescent spectral characteristics of the oxide fluorescent material forming the wavelength converting member 22. An excitation region of an excitation spectrum is defined as a region where the excitation intensity is half or more of a peak value. A visible light region is defined as a wavelength region of 380 nm to 780 nm.

An absorption region of the oxide fluorescent material in the visible light region, which is the wavelength region of 380 nm to 780 nm, is a wavelength region of about 420 nm to 520 nm. A yellow fluorescent spectrum (the first fluorescence) has a broad spectrum in which a fluorescent peak is present at a center wavelength of 575 nm ($\lambda 3$) and a half-value width is 95 nm. A waveform of the fluorescent spectrum has characteristics that a long wavelength side is gently tilted to the fluorescent peak wavelength as compared with a short wavelength side.

The oxide fluorescent material has a low excitation intensity to the laser light (the first excitation light) of the blue-violet color of the center wavelength of 415 nm ($\lambda 1$), which is the primary light emitted from the first laser diode 5, and is not excited. It can be considered that the oxide fluorescent material is not excited, when the excitation intensity is about 1/3 or less of an intensity to the blue laser light (the second excitation light) with the wavelength of 445 nm ($\lambda 2$), which is the primary light emitted from the second laser diode 6.

The wavelength converting member 22 is formed by dispersing a fluorescent material in a powder form in a sealing material such as silicone resin or glass and solidifying the sealing material. A thickness of the wavelength converting member 22 or a concentration condition of the powder fluorescent material to be mixed in the sealing material is set in consideration of fluorescent material excitation light absorptivity or wavelength conversion efficiency characteristics, etc., so that a part of the blue laser light entering as the primary light from the third optical fiber 11, in other words, the blue laser light emitted from the second laser diode 6 (the center wavelength of 445 nm: the second excitation light source: $\lambda 2$), is converted into a desired wavelength here, i.e., the yellow fluorescence ($\lambda 3$).

The wavelength converting member 22 is disposed on an optical path axis of the blue-violet laser light (the first excitation light) or the blue laser light (the second excitation light) of the primary light entering from the third optical fiber 11 connected to the entrance portion 24 of the holder 20. The wavelength converting member 22 is formed into, for example, a columnar shape. The wavelength converting member 22 is installed in the vicinity of an optical axis of the first or second excitation light from the third optical fiber 11.

The system control section 4 is connected to an input section (a mode instructing section) 13. The input section 13 sends, to the system control section 4, observation mode information M indicating one of observation modes such as a normal light observation mode and a special light observation mode as a specific observation mode in which observation is executed. For example, the input section 13 receives a manual operation of an operator, etc. to input the observation mode information M, or inputs the observation mode information M from an external computer.

The image acquiring device 3 performs, by the color regions, color separation of an image of an irradiated region S of the subject Q that is the observation target irradiated with the illuminating light P by the light source device 2, to acquire the image. The image acquiring device 3 images reflected light from the subject Q to acquire the image of the subject Q, e.g., a normal light observation image in the normal light observation mode or a special light observation image in the special light observation mode. The image acquiring device 3 includes the imaging section 30, an image processing section 31, and an image display section 35.

In the imaging section 30, imaging is controlled by an imaging control signal PC output from the system control section 4. The imaging section 30 images reflected light F from the irradiated region S of the subject Q with the illuminating light P emitted from the light source device 2, and outputs a pixel signal of each of BGR pixels. The imaging section 30 includes, for example, an imaging device such as a CCD imager in which an imaging element such as a CCD is used, or a CMOS imager.

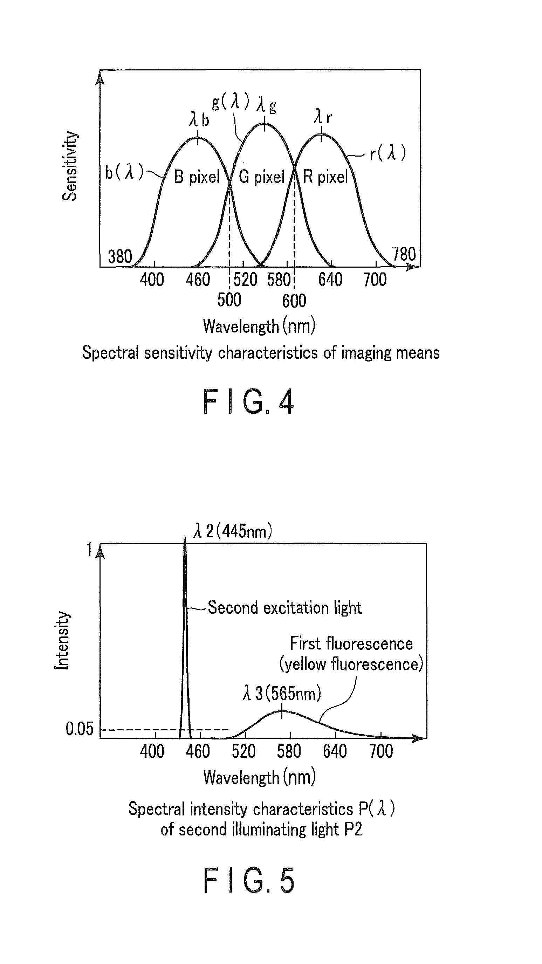

FIG. 4 shows sensitivity wavelength characteristics of a typical CCD imager. The CCD imager includes the B pixels having a sensitivity peak at a wavelength of 460 nm ($\lambda$b) in the blue region, the G pixels having a sensitivity peak at a wavelength of 540 nm ($\lambda$g) in the green region, and the R pixels having a sensitivity peak at a wavelength of 630 nm ($\lambda$r) in the red region.

A sensitivity region of the B pixel is present up to the wavelength of 540 nm on the long wavelength side. A sensitivity region of the R pixel is present up to the wavelength of 540 nm on the short wavelength side. Therefore, the B pixel and the G pixel as well as the G pixel and the R pixel include a wavelength region where sensitivities of the adjacent wavelength regions overlap.

Light receiving sensitivity characteristics of the B pixel to a wavelength $\lambda$ are defined as $b(\lambda)$, light receiving sensitivity characteristics of the G pixel are defined as $g(\lambda)$, and light receiving sensitivity characteristics of the R pixel are defined as $r(\lambda)$.

The image processing section 31 performs image processing of each pixel signal of each of the B pixels, the G pixels, and the R pixels output from the imaging section 30, and acquires the normal light observation image or the special light observation image of the subject Q. The image processing section 31 includes a first frame memory 32, a second frame memory 33, and a calculator 34.

The image acquiring section 31 acquires the special light observation image on the basis of the illuminating light output from the light source device 2 and wavelength characteristics of the color regions of the image acquiring section 31, when there is an instruction of the special light observation mode. The special light observation image is acquired by generating information of a narrowband spectrum element by an overlap of wavelength characteristics of the illuminating light such as the first and second illuminating light P1 and P2 with the wavelength characteristics of the color regions of the image acquiring section 31, and by combining the information of the narrowband spectrum element with reflected images acquired every irradiation with the first and second illuminating lights P1 and P2.

The image acquiring section 31 acquires information of the first narrowband spectrum element from the reflected image of each first imaging frame acquired every time the subject Q is irradiated with the first illuminating light P1 from the light source device 2, acquires information of a second narrowband spectrum element from the reflected image of each second imaging frame acquired every time the subject Q is irradiated with the second illuminating light P2 from the light source device 2, and combines the information of the first narrowband spectrum element with the information of the second narrowband spectrum element to construct the special light observation image.

The first narrowband spectrum element and the second narrowband spectrum element include wavelengths each having a high absorption intensity of the subject Q and belonging to different absorption peaks, respectively.

Each of the first and second frame memories 32 and 33 successively takes therein the pixel signal of each of the B pixels, the G pixels, and the R pixels output from the imaging section 30 and stores the signal as the reflected light image of each frame. The storage of the reflected light image of each frame into the first and second frame memories 32 and 33 is controlled by a storage frame signal FM output from the system control section 4.

The calculator 34 reads out each reflected light image stored in the first frame memory 32 and the second frame memory 33, respectively, and performs calculation to each reflected light image to generate image information to be displayed in the image display section 35, e.g., the normal light observation image or the special light observation image.

The image display section 35 displays the normal light observation image or the special light observation image acquired by the image processing of the image processing section 31. The image display section 35 specifically includes, e.g., a CRT, a liquid crystal display, etc.

When the observation mode information M indicating the normal light observation mode or the special light observation mode is input from the input section 13, the system control section 4 transmits a light source control signal LC to the light source control section 7, transmits the imaging control signal PC to the imaging section 30, and transmits the storage frame signal FM to the image processing section 31, in accordance with contents of the observation mode information M.

Specifically, the system control section 4 transmits the light source control signal LC to the light source control section 7 so that the second laser diode 6 is lit, in the normal light observation mode. Additionally, the system control section 4 transmits, to the imaging section 30, the imaging control signal PC to repeat, in the imaging section 30, exposure processing and transmission of the pixel signal of each of the BGR pixels which can be obtained by the exposure processing. The system control section 4 transmits, to the image processing section 31, the storage frame signal FM indicating a storage destination to store, in the second frame memory 33, the reflected light image of each frame which is obtained by the imaging of the imaging section 30.

Therefore, in the normal light observation mode, the image processing section 31 stores, in the second frame memory 33, the reflected light image of each frame which is obtained by the imaging of the imaging section 30, when the second laser diode 6 is lit.

The calculator 34 reads out the reflected light image stored in the second frame memory 33, performs calculation to the reflected light image to generate the normal light observation image of the subject Q when the subject Q is irradiated with the white light, and displays the normal light observation image in the image display section 35.

On the other hand, in the special light observation mode, the system control section 4 successively lights the primary light sources in the light source device 2, i.e., the first and second laser diodes 5 and 6 to successively irradiate the subject Q that is the observation target with the illuminating light P of the primary light of the wavelengths. The system control section 4 transmits the light source control signal LC to the light source control section 7 so that the first and second laser diodes 5 and 6 are alternately lit. Additionally, the system control section 4 transmits, to the imaging section 30, the imaging control signal PC to repeat, to the imaging section 30, the exposure processing and the transmission of the pixel signal of each of the BGR pixels in the same manner as described above. The system control section 4 transmits, to the image processing device 31, the storage frame signal FM indicating the storage destination to alternately store, in the first and second frame memories 32 and 33, the reflected light image of each frame which is obtained by the imaging of the imaging section 30.

Therefore, in the special light observation mode, the image processing section 31 successively and repeatedly stores the reflected light image of each frame which is obtained by the imaging of the imaging section 30 alternately in the first and second frame memories 32 and 33 in synchronization with the alternate lighting of the first and second laser diodes 4 and 5, when the first and second laser diodes 4 and 5 are alternately lit. In the first frame memory 32, the reflected light image is stored as information including the information of the first narrowband spectrum element. In the second frame memory 33, the reflected light image is stored as information including the information of the second narrowband spectrum element.

The calculator 34 reads out the reflected light images stored in both of the first and second frame memories 32 and 33, performs calculation to each reflected light image to generate the special light observation image, and displays the special light observation image in the image display section 35.

In the special light observation mode, the reflected light image obtained by the imaging of the imaging section 30 is successively and repeatedly stored alternately in the first frame memory 32 and the second frame memory 33 for each frame. Thus, when the storage destination is indicated as the first frame memory 32 by the storage frame signal FM output from the system control section 4, the reflected light image to be stored in the first frame memory 32 is generated in the imaging section 30 in a period of the exposure processing performed immediately before. Immediately after the exposure processing, the reflected light image is stored in the first frame memory 32.

Similarly, when the storage frame signal FM indicates the second frame memory 33 as the storage destination, the reflected light image to be stored in the second frame memory 33 is generated in the imaging section 30 in the period of the exposure processing performed immediately before. Immediately after the exposure processing, the reflected light image is stored in the second frame memory 33.

The image processing section 31 stores the reflected light image of each frame in the first frame memory 32 or the second frame memory 33 in accordance with the storage frame signal FM transmitted from the system control section 4 as described above. The present invention is not limited to this example, and the image processing section 31 may directly input the observation mode information M indicating the normal light observation mode or the special light observation mode from the input section 13 to store the reflected light image in the first frame memory 32 or the second frame memory 33. For example, on inputting the observation mode information M indicating the normal light observation, the image processing section 31 stores, in the second frame memory 33, the reflected light image of each frame which is obtained by the imaging of the imaging section 30, when the second laser diode 6 is lit.

On inputting the observation mode information M indicating the special light observation mode, the image processing section 31 successively and repeatedly stores the reflected light image obtained by the imaging of the imaging section 30 alternately in the first frame memory 32 and the second frame memory 33 for each frame, when the first and second laser diodes 4 and 5 are alternately lit. As to the storage destination of the reflected light image, the image processing section 31 judges whether or not the exposure processing is performed in a period when one of the first and second laser diodes 4 and 5 is lit, and one of the first and second frame memories 32 and 33 in which the image is to be stored may be determined in accordance with this judgment result.

In the special light observation mode, the image processing section 31 acquires the reflected light images every time the subject Q that is the observation target is irradiated with the respective illuminating light to successively and repeatedly store the images alternately in the first frame memory 32 and the second frame memory 33 for each frame, and generates the special light observation image of the subject Q on the basis of each reflected light image.

The image processing section 31 has white balance coefficients to determine color information during the image generation in the normal light observation mode and during the image generation in the special light observation mode. The white balance coefficients are set by use a white plate having essentially flat reflection characteristics in the visible light region to, for example, sensitivity characteristics in which light emission intensity characteristics $P(\lambda)$ to the wavelength $\lambda$ of the illuminating light P are multiplied by the light receiving sensitivity characteristics $b(\lambda)$, $g(\lambda)$, and $r(\lambda)$ of the CCD as the imaging element of the imaging section 30.

Color component B, G, and R in which the light emission intensity characteristics $P(\lambda)$ of the illuminating light P are multiplied by the light receiving sensitivity characteristics $b(\lambda)$, $g(\lambda)$, and $r(\lambda)$ of the CCD, respectively, are calculated as follows.

[Equation 1]

$$B = \int_{380}^{780} P(\lambda) \bullet b(\lambda)$$

$$G = \int_{380}^{780} P(\lambda) \bullet g(\lambda)$$

$$R = \int_{380}^{780} P(\lambda) \bullet r(\lambda)$$

For example, when the reflection characteristics (absorption characteristics) of the white plate are constant in the visible light region, a white balance coefficient Wb of the color component B to the color component G is B/G, and a white balance coefficient Wr of the color component R to the color component G is R/G.

In a case where the respective white balance coefficients Wb and Wr are 1.0, it indicates that a blue component, a green component, and a red component are well-balanced when spectral characteristics of the illuminating light P are multiplied by the sensitivity characteristics of the respective pixels of the CCD.

According to correction by the white balance coefficients Wb and Wr, it is possible to generate the normal light observation image and the special light observation image, even when the spectrum of the illuminating light P is different from the white light. When the white balance coefficients Wb and Wr are excessively small or large numeric values, noise of a specific pixel is amplified during color balance setting, and noise of the generated image enlarges, so that the white balance coefficients Wb and Wr are preferably in a range of, e.g., 1/3 to 3.

Therefore, concerning ratios of the color components in the illuminating light P, a light quantity of an R color with a wavelength of 600 nm or more to which the R pixel has a maximum sensitivity is preferably 1/3 or more of a light quantity with a wavelength region of, e.g., 525 nm to 555 nm to which the G pixel has a maximum sensitivity.

The light source control section 7 inputs the light source control signal LC output from the system control section 4, and controls the lighting of the first or second laser diode 5 or 6 in accordance with the light source control signal LC. For example, in the normal light observation mode, the system control section 4 transmits the light source control signal LC to light the second laser diode 6, therefore, the light source control section 7 lights and drives the second laser diode 6.

On the other hand, in the special light observation mode, the system control section 4 transmits the light source control signal LC so that the first and second laser diodes 5 and 6 are alternately lit, and hence, the light source control section 7 alternately lights and drives the first and second laser diodes 5 and 6.

The light source control section 7 performs control of drive currents or a drive system, e.g., driving such as pulse driving or continuous driving (CW) of the first and second laser diodes 5 and 6 to light the first and second laser diodes 5 and 6.

Next, an operation of the device having such a structure as described above will be described.

(1) Case where the normal light observation mode is input as the observation mode information M

When the observation mode of the normal light observation mode is input into the input section 13 from a manual operation of an operator or the like or an external computer, the input section 13 sends the observation mode information M indicating the normal light observation mode to the system control section 4.

On inputting the observation mode information M indicating the normal light observation mode, the system control section 4 transmits, to the light source control section 7, the light source control signal LC to light the second laser diode 6. In addition, the system control section 4 transmits, to the imaging section 30, the imaging control signal PC to repeat, in the imaging section 30, the exposure processing and the transmission of the pixel signal of each of the BGR pixels which is obtained by the exposure processing. The system control section 4 transmits, to the image processing section 31, the storage frame signal FM indicating the storage destination to store, in the second frame memory 33, the reflected light image of each frame which is obtained by the imaging of the imaging section 30.

On inputting the light source control signal LC to light the second laser diode 6, the light source control section 7 lights and drives the second laser diode 6 in accordance with the light source control signal LC. The second laser diode 6 emits the primary light (the first excitation light) that is the blue laser light of the center wavelength of 445 nm ($\lambda 2$). The blue laser light is guided by the second optical fiber 10, the optical coupler 9, and the third optical fiber 11 to enter the wavelength conversion unit 12.

The wavelength conversion unit 12 converts the wavelength of a part of the blue laser light entered in the unit to that of the yellow fluorescence ($\lambda 3$) by the fluorescent material that is the wavelength converting member 22. In addition, the wavelength conversion unit 12 transmits the blue laser light which is not absorbed by the fluorescent material and whose wavelength is not converted to that of the yellow fluorescence. As a result, the wavelength conversion unit 12 emits the yellow fluorescence whose wavelength has been converted and the blue laser light as the illuminating light P (the second illuminating light P2) from the emission end. The subject Q is irradiated with the second illuminating light P2, and the irradiated region S is formed on the subject Q.

The second illuminating light P2 emitted from the wavelength conversion unit 12 has a predetermined light distribution angle. A size of the irradiated region S of the second illuminating light P2 with which the subject Q is irradiated is formed in accordance with a relation between the light distribution angle and a distance from the emission end of the wavelength conversion unit 12 to the subject Q. The irradiated region S is a region of the size including at least a range in which the imaging is to be performed by the imaging section 30, and is preferably broader than the above imaging range of the imaging section 30.

The second illuminating light P2 in the normal light observation mode is preferably white light. To observe the subject Q having an ivory color to a red color that is especially present in biological tissue as the subject Q, a flat spectral component continuous over a wavelength region from a green color to the red color is considered to be effective.

FIG. 5 shows the spectral characteristics $P(\lambda)$ of the second illuminating light P2. A region having the spectral component in each of the color regions of BGR is defined as a region where a light intensity larger than, e.g., 1/20 of a maximum light intensity of each of the color regions of BGR is present. On the other hand, it is defined that the light intensity of 1/20 or less of the maximum light intensity does not have any spectral components.

The second illuminating light P2 emitted from the wavelength conversion unit 12 is mixed light of the blue laser light of a narrowband having a peak at the center wavelength of 445 nm ($\lambda 1$) and the yellow fluorescence having a peak in the vicinity of a center wavelength of 565 nm ($\lambda 3$). The mixed light is set to a component ratio between the blue laser light and the yellow fluorescence so that the light approximates white light.

In the second illuminating light P2, the maximum intensity of a wavelength region of 450 nm to 480 nm between the blue laser light and the yellow fluorescence is 1/20 or less of the intensity of the peak wavelength ($\lambda 1$) of the blue laser light.

A light quantity with a wavelength region of 450 nm to 480 nm is 1/20 or less of a light quantity of a wavelength region of 400 nm to 440 nm.

A ratio of the light quantities in the blue region, the green region, and the red region of the second illuminating light P2 is about 5 (blue): 3 (green): 2 (red). A difference between the wavelength $\lambda 2$ (the blue laser light) and the wavelength $\lambda 3$ (the yellow fluorescence) is about 150 nm. A wavelength difference between a half-value width end of the blue laser light and a half-value width end of the yellow fluorescence is about 80 nm.

On the other hand, the imaging section 30 images the reflected light F from the irradiated region S of the subject Q irradiated with the second illuminating light P2, and outputs the pixel signal of each of the BGR pixels.

When the normal light observation mode is input as the observation mode information M, the image processing section 31 follows the storage frame signal FM output from the system control section 4 and indicating the storage destination to store, in the second frame memory 33, the reflected light image of each frame, inputs the respective pixel signals of BGR acquired by the imaging of the imaging section 30, generates the reflected light image for each frame on the basis of a pixel signal level of each of BGR, and stores the reflected light image for each frame in the second frame memory 33.

The calculator 34 of the image processing section 31 performs calculation to the reflected light image stored in the second frame memory 33 to generate the normal light observation image of the subject Q when the subject Q is irradiated with the white light, and displays the normal light observation image in the image display section 35, e.g., the CRT, the liquid crystal display or the like. Specifically, the calculator 34 subjects a light receiving signal of each of the BGR pixels that is stored in the second frame memory 33 to predetermined image processing such as white balance, noise reduction, structure emphasis, and gamma correction image processing, and generates the normal light observation image. The normal light observation image is generated by using all spectral components included in the second illuminating light P2.

(2) Case where the special light observation mode is input as the observation mode

When the observation mode of the special light observation mode is input into the input section 13 from the manual operation of the operator, etc., or the external computer, the input section 13 sends the observation mode information M indicating the special light observation mode to the system control section 4.

On inputting the observation mode information M indicating the special light observation mode, the system control section 4 transmits, to the light source control section 7, the light source control signal LC to alternately light the first and second laser diodes 5 and 6. The light source control signal LC includes information that distinguishes one of the first and second laser diodes 5 and 6 to be lit.

Therefore, the system control section 4 alternately transmits, e.g., the light source control signal LC to light the first laser diode 5 and the light source control signal LC to light the second laser diode 6. In addition, the system control section 4 transmits, to the imaging section 30, the imaging control signal PC to repeat the exposure processing and the transmission of the pixel signal of each of the BGR pixels in the same manner as described above to the imaging section 30. The system control section 4 transmits, to the image processing section 31, the storage frame signal FM indicating the storage destination to store the reflected light image of each frame which is obtained by the imaging of the imaging section 30 alternately in the first and second frame memories 32 and 33.

The system control section 4 alternately transmits, e.g., the light source control signal LC to light the first laser diode 5 and the light source control signal LC to light the second laser diode 6.

Into the light source control section 7, the light source control signal LC to light the first laser diode 5 and the light source control signal LC to light the second laser diode 6, which are transmitted from the system control section 4, are alternately input to alternately light and drive the first and second laser diodes 5 and 6 in accordance with the respective light source control signals LC.

When the first laser diode 5 is lit, the first laser diode 5 emits, as the primary light, the blue-violet laser light of a narrowband in which a half-value width is several nm or less, at the center wavelength of 415 nm ($\lambda$1) that becomes the light emission peak. The blue-violet laser light is guided by the second optical fiber 10, the optical coupler 9, and the third optical fiber 11 to enter the wavelength conversion unit 12.

The wavelength converting member 22 has a low excitation intensity to the blue-violet laser light (the center wavelength of 415 nm: $\lambda$1) emitted from the first laser diode 5, and is not excited. Therefore, the wavelength conversion unit 12 transmits the blue-violet laser light ($\lambda$1), and emits the blue-violet laser light ($\lambda$1) as the illuminating light P (the first illuminating light P1).

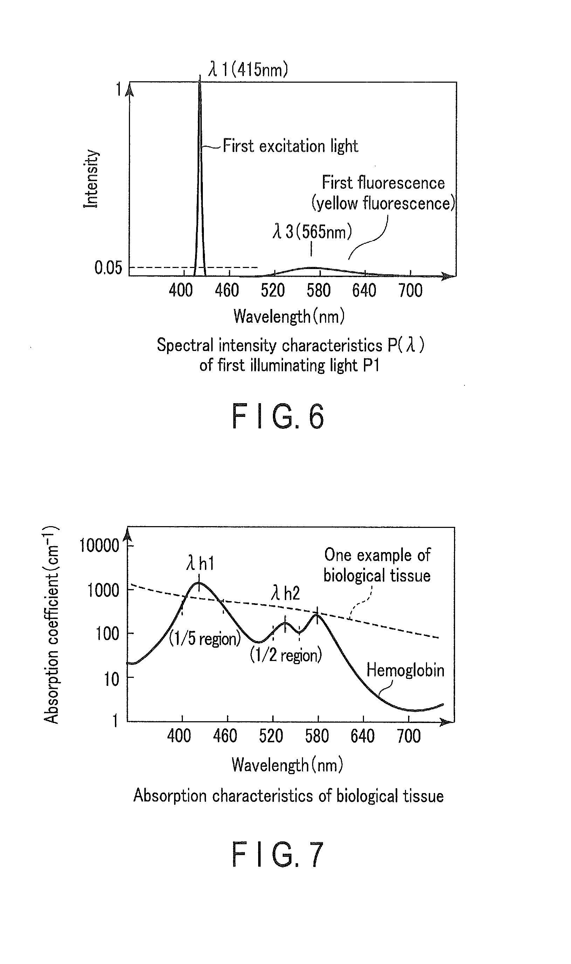

FIG. 6 shows the spectral characteristics P ($\lambda$1) of the first illuminating light P1. The light intensity of the yellow fluorescence emitted when the wavelength converting member 22 is irradiated with the first illuminating light P1 is 1/20 or less of the maximum light intensity of the blue-violet laser light (the center wavelength of 415 nm: $\lambda$1). As described above, the wavelength converting member 22 has a low excitation intensity to the blue-violet laser light emitted from the first laser diode 5, and is not excited.

The imaging section 30 images the reflected light F from the irradiated region S of the subject Q irradiated with the first illuminating light P1, and outputs the pixel signal of each of the BGR pixels.

In the special light observation mode, the image processing section 31 inputs the pixel signal of each of the BGR pixels acquired by the imaging of the imaging section 30 in accordance with the storage frame signal FM output from the system control section 4 and indicating the storage destination to store the reflected light image of each frame in the first frame memory 32, at a lighting timing when the first laser diode 5 is lit. The image processing section 31 generates the reflected light image of one frame on the basis of each of the BGR pixel signal levels, and stores the reflected light image of the frame in the first frame memory 32.

On the other hand, when the second laser diode 6 is lit, the second laser diode 6 emits the blue laser light of the center wavelength of 445 nm ($\lambda$2) in the same manner as described above. The blue laser light is guided by the second optical fiber 10, the optical coupler 9, and the third optical fiber 11 to enter the wavelength conversion unit 12.

The wavelength conversion unit 12 converts the wavelength of a part of the blue laser light entered in the unit to that of the yellow fluorescence ($\lambda$3) by the fluorescent material that is the wavelength converting member 22. In addition, the wavelength conversion unit 12 transmits the blue laser light which is not absorbed by the fluorescent material and whose wavelength is not converted to that of the yellow fluorescence. Therefore, the wavelength conversion unit 12 emits the yellow fluorescence and the blue laser light as the second illuminating light P2. The subject Q is irradiated with the second illuminating lights P2, and the irradiated region S is formed on the subject Q.

The imaging section 30 images the reflected light F from the irradiated region S of the subject Q irradiated with the second illuminating light P2, and outputs the pixel signal of each of the BGR pixels.

In the special light observation mode, the image processing section 31 inputs the respective pixel signals of BGR acquired by the imaging of the imaging section 30 in accordance with the storage frame signal FM output from the system control section 4 and indicating the storage destination to store the reflected light image of each frame in the second frame memory 33, at the lighting timing when the second laser diode 6 is lit. The image processing section 31 generates the reflected light image of each frame on the basis of each BGR pixel signal level, and stores the reflected light image of each frame in the second frame memory 33.

Subsequently, in the special light observation mode, the first and second laser diodes 4 and 5 are alternately, successively and repeatedly lit, and hence, when the first laser diode 5 is lit, the reflected light image of the subject Q is stored in the first frame memory 32. When the second laser diode 6 is lit, the reflected light image of the subject Q is stored in the second frame memory 33. That is, when the first laser diode 5 is lit, the reflected light image generated in an exposure period of the imaging section 30 is stored in the first frame memory 32 at the lighting timing. When the second laser diode 6 is lit, the reflected light image generated in the exposure period of the imaging section 30 is stored in the second frame memory 33 at the lighting timing.

Next, an operation in which a high-contrast special light image can be obtained will be described.

In the subject Q, at least two type of tissues which are different in light absorption characteristics, e.g., the blood vessel K and a biological tissue such as a mucous membrane, are present.

FIG. 7 shows an absorption coefficient that becomes an index of the absorption intensity of hemoglobin flowing in the blood vessel K of the biological tissue. In the visible light region of a wavelength region of 380 nm to 780 nm, hemoglobin has absorption intensity peaks in respective different wavelengths, i.e., in the vicinity ($\lambda$h1) of the wavelength of 415 nm and the vicinity ($\lambda$h2) of the wavelength of 540 nm. The absorption intensity in the vicinity of the wavelength of 415 nm ($\lambda$h1) has the largest property.

Generally in an NBI observation which is a special light observation by an endoscope or the like, two wavelength light including the respective wavelength regions in the vicinity ($\lambda$h1) of the wavelength of 415 nm and the vicinity ($\lambda$h2) of the wavelength of 540 nm, e.g., the light of the wavelength region of about 400 nm to 440 nm and the light of the wavelength region of about 525 nm to 555 nm are used as the illuminating light P (the observation lights). The NBI observation is a technology (the special light observation) which utilizes the depth of the light penetration from the surface of biological tissue and the fact that scattering characteristics of the light of respective wavelengths have properties different from each other, and which the blood vessel K or the like is observed with high contrast, thereby making it easy to find a cancer, etc.

In the first illuminating light P1 of the present embodiment, the blue-violet laser light of the center wavelength of 415 nm ($\lambda$1) is the primary light, and this blue-violet laser light is absorbed and scattered in a comparative surface layer portion of the biological tissue, and is therefore effective for the observation of the blood vessel K in the vicinity of the surface of the biological tissue that is the subject Q.

The second illuminating light P2 includes a component that becomes the white light by the blue laser light of the center wavelength of 445 nm ($\lambda$2) and the yellow fluorescence ($\lambda$3) having the peak in the vicinity of the wavelength of 565 nm. The yellow fluorescence is a broad spectrum and includes the light in the vicinity of the wavelength of 540 nm.

The light in the vicinity of the wavelength of 540 nm, which is included in the yellow fluorescence, is scattered to a certain degree during the irradiation of the biological tissue, but travels deeper under the skin of the biological tissue than the light with the wavelength of 415 nm ($\lambda$1), and is absorbed and scattered in the blood vessel K or the like which is present under the skin, so that the light is effective for the observation of the blood vessel K of a subcutaneous tissue. Preferably, the peak wavelength $\lambda$3 of the components of the yellow fluorescence is present in a wavelength band of the absorption coefficient that is 1/2 or greater than the absorption coefficient of the hemoglobin absorption peak wavelength of 540 nm of the green region, which results in a direction of increase of a ratio at which the light is absorbed in the observation of the blood vessel K of the subcutaneous tissue and increase of the sensitivity of the G pixel, so that a high-contrast image can be obtained.

In the meantime, the absorption characteristics of hemoglobin indicate the tendency that the absorption intensity rapidly lowers from the vicinity ($\lambda$h1) of the wavelength of 415 nm toward the long wavelength side. For example, when the absorption coefficient of the wavelength of 450 nm is compared with that of the wavelength of 415 nm ($\lambda$h1), the absorption intensity lowers down to about 1/5 with a wavelength difference of 35 nm.

On the other hand, the biological tissue in the subject Q often displays an ivory color to a red color. For example, as shown in an example in FIG. 7, a certain tissue is also present in which the absorption coefficient gradually lowers from the blue region toward the red region, is smaller than the absorption coefficient of hemoglobin in the vicinity ($\lambda$h1) of the wavelength of 415 nm of the blue region, and is larger than the absorption coefficient of hemoglobin in the vicinity of the wavelength of 450 nm.

To observe the blood vessel K with a high contrast, it is necessary to increase the ratio of the light quantity of the blue region where hemoglobin is absorbed in the biological tissue in regards to the blue laser light of the center wavelength of 445 nm ($\lambda$2) included as the observation light in the second illuminating light P2. For this purpose, a first light quantity of the vicinity ($\lambda$h1) of the wavelength of 415 nm in which the absorption coefficient of hemoglobin is larger than the absorption coefficient of the biological tissue is made larger than a second light quantity of the vicinity of the wavelength of 450 nm in which the above absorption coefficient of hemoglobin is smaller than the absorption coefficient of the biological tissue.

In the B pixels of the CCD of the imaging section 30 for use in the image acquisition, the sensitivity in the vicinity of the wavelength of 450 nm is about twice as high as that in the vicinity of the wavelength of 415 nm ($\lambda$1) in which the absorption coefficient of hemoglobin is larger than the absorption coefficient of the biological tissue as described above. Consequently, when the light quantity in the wavelength region of 450 nm to 480 nm which is the blue region from the wavelength of 450 nm to the long wavelength side is about 1/2 of the light quantity in the wavelength region of 400 nm to 440 nm in the vicinity of the wavelength of 415 nm, the sensitivity of the B pixel is about twice as high as that described above. As a result, the light quantity in the wavelength region of 450 nm to 480 nm becomes larger than the light quantity of the wavelength of 415 nm ($\lambda$1). Therefore, the light quantity in the wavelength region of 450 nm to 480 nm (the wavelength in the vicinity of the blue laser light with a wavelength of 445 nm ($\lambda$2)) has an influence as image noise when the contrast is heightened. That is, during the observation of the blood vessel K (in which hemoglobin flows) in the vicinity of the surface of the subject Q, the influence of the image noise generated by the absorption into the biological tissue is received.

For reduction of the image nose, it is effective to decrease the light quantity in the wavelength region of 450 nm to 480 nm to 1/5 or less of the light quantity in the wavelength region of 400 nm to 440 nm. When the light quantity in the wavelength region of 450 nm to 480 nm is set to 1/10 or less of the light quantity in the wavelength region of 400 nm to 440 nm, the high-contrast image can be obtained in addition to the image nose reduction.

Next, an operation of generating the special light observation image based on the reflected light image stored in the first frame memory 32 when the subject is irradiated with the first illuminating light P1 and the reflected light image stored in the second frame memory 33 when the subject is irradiated with the second illuminating light P2 will be described.

When the subject Q is irradiated with the first illuminating light P1 or the second illuminating light P2, a part of each of the first illuminating light P1 and the second illuminating light P2 is absorbed by the absorption characteristics of the blood vessel K or the biological tissue present in the irradiated region S on the subject Q, and the remaining part of the light is scattered and reflected to be received by the B pixels, the G pixels, and the R pixels of the imaging section 30.

The light receiving sensitivity characteristics of the B pixel are present in the wavelength region of 380 nm to 540 nm. The reflected light F received by the B pixels when the subject is irradiated with the second illuminating light P2 corresponds to the blue laser light ($\lambda$2) that is the second primary light and a short wavelength region portion of the yellow fluorescence ($\lambda$3). Additionally, the B pixel has a low sensitivity in the vicinity of the wavelength of 520 nm, and thus, the component received by the B pixels mainly becomes the blue laser light.

The blue laser light ($\lambda$2) that is the primary light included in the second illuminating light P2 causes the image noise in the special light observation as described above.

The reflected light F received by the B pixels when the subject is irradiated with the first illuminating light P1 becomes the blue-violet laser light ($\lambda$1) that is the primary light. The component received by the B pixels becomes the blue-violet laser light. The blue-violet laser light ($\lambda$1) has an intensity in the vicinity ($\lambda$h1) of the wavelength of 415 nm which is a first absorption peak of hemoglobin as described above, and is absorbed and scattered in the comparative surface layer portion of the biological tissue.

Consequently, in the reflected light image of each frame which is acquired by irradiating the subject with the first illuminating light P1 and stored in the first frame memory 32, information on a status of the blood vessel K in the vicinity of the surface of the tissue is stored as the information of the B pixels. In the same reflected light image of each frame which is acquired by irradiating the subject with the first illuminating light P1, information of the reflected light F of the primary light, i.e., the blue laser light ($\lambda$2) included in the second illuminating light P2, is not included as the information of the B pixels, and the influence of the noise generated by the absorption of the blue laser light by the biological tissue other than hemoglobin can be reduced.

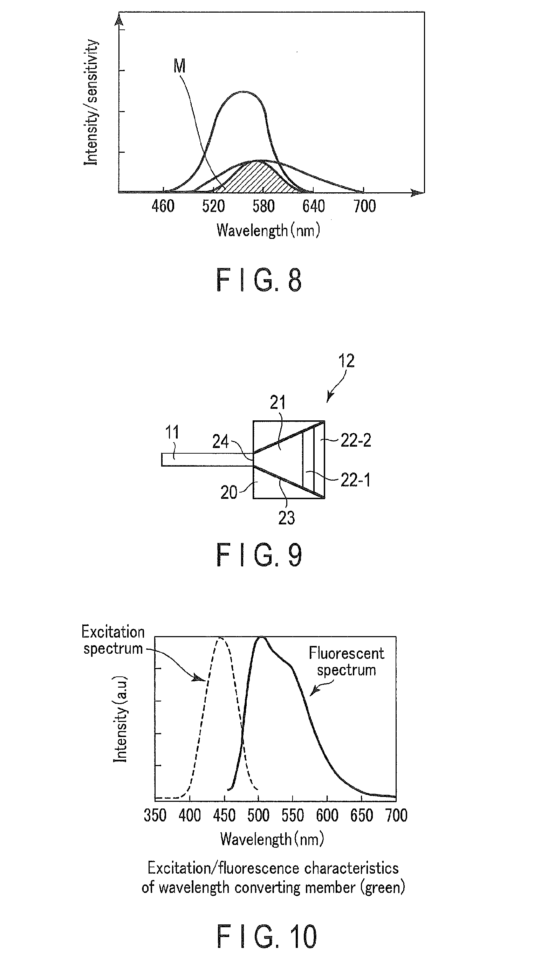

FIG. 8 shows a part M corresponding to the second narrowband spectrum element in which the light receiving sensitivity characteristics of the G pixel overlap with the wavelength spectrum of the yellow fluorescence ($\lambda$3). The light receiving sensitivity characteristics of the G pixel are present in a wavelength region of 460 nm to 640 nm. The reflected light F received by the G pixels when the subject is irradiated with the second illuminating light P2 corresponds to the wavelength region in the vicinity of the intensity peak of the yellow fluorescence ($\lambda$3). The part M in which the light receiving sensitivity characteristics of the G pixel overlap with the wavelength spectrum of the yellow fluorescence ($\lambda$3) is taken into the G pixels.

A short wavelength end of the intensity spectrum of the yellow fluorescence ($\lambda$3) having the peak in the vicinity of the wavelength of 565 nm is held in the light receiving sensitivity characteristics of the G pixel. As shown in FIG. 8, the part M in which the light receiving sensitivity characteristics of the G pixel overlap with the wavelength spectrum of the yellow fluorescence ($\lambda$3) has characteristics narrower than each wavelength spectrum, and corresponds to the second narrowband spectrum element.

The part M of the second narrowband spectrum element has the intensity in the vicinity ($\lambda$h2) of the wavelength of 540 nm which is a second absorption peak of hemoglobin. In the part M of the second narrowband spectrum element, the light of the wavelength is scattered to a certain degree during the irradiation of the biological tissue as described above, but travels deeper under the skin than the light with a wavelength of 415 nm ($\lambda$h1), and is absorbed and scattered in the blood vessel K or the like which is present under the skin, so that the light is effective for the observation of the blood vessel K of the subcutaneous tissue.

When the subject is irradiated with the first illuminating light P1, the first illumination light P1 does not have the intensity in the wavelength region having the light receiving sensitivity of the G pixel, and therefore the G pixels do not receive the reflected light F.

A long wavelength boundary value of the G pixel is present between a short wavelength boundary value and a long wavelength boundary value of the yellow fluorescence ($\lambda$3), and a short wavelength boundary value of the G pixel is present at a shorter wavelength than the short wavelength boundary value of the yellow fluorescence (λ3). The present invention is not limited to this example, the short wavelength boundary value of the G pixel may be present between the short wavelength boundary value and the long wavelength boundary value of the yellow fluorescence (λ3), and the long wavelength boundary value of the G pixel may be present at a longer wavelength than the long wavelength boundary of the yellow fluorescence.

On the other hand, the light receiving sensitivity characteristics of the R pixel are present in a wavelength region of 540 nm to 720 nm. The long wavelength region of the yellow fluorescence (λ3) of the second illuminating light P2 also includes a red region component with a wavelength of 580 nm or more. Therefore, the R pixels mainly receive the red region component of the second illuminating light P2. When the subject is irradiated with the first illuminating light P1, the R pixels do not have the light receiving sensitivity intensity level of the wavelength region included in the first illuminating light P1, and therefore do not receive the reflected light F.

The respective BGR pixel signals received by the CCD of the imaging section 30 are transmitted to the image processing section 31. When the special light observation mode is input as the observation mode information M, the image processing section 31 follows the storage frame signal FM output from the system control section 4, generates the reflected light image of each corresponding frame to store the image in the first frame memory 32 every time the subject Q is irradiated with the first illuminating light P1, and generates the reflected light image of each corresponding frame to store the image in the second frame memory 33 every time the subject Q is irradiated with the second illuminating light P2. The storage frame signal FM indicates the storage destination to successively store the reflected light image of each frame alternately in the first and second frame memories 32 and 33. The first and second illuminating light P1 and P2 are alternately and successively lit.

Specifically, in the special light observation mode, the image processing section 31 stores the pixel signal output from the B pixels of the imaging section 30 in the first frame memory 32 every time the subject Q is irradiated with the first illuminating light P1 of the blue-violet laser light (λ1). The image processing section 31 stores the pixel signal output from the G pixels of the imaging section 30 in the second frame memory 33 every time the subject Q is irradiated with the second illuminating light P2 of the narrowband blue laser light (λ2).

The image processing section 31 reads out the pixel signal of the B pixels which is stored in the first frame memory 32, reads out the pixel signal of the G pixels which is stored in the second frame memory 33, and sends the pixel signal of the B pixels and the pixel signal of the G pixels to the calculator 34.

The calculator 34 assigns the pixel signal of the B pixels which is read out from the first frame memory 32 to the B pixel and the G pixels of the image information to be sent to the image display section 35, assigns the pixel signal of the G pixels which is read out from the second frame memory 33 to the R pixels of the image information to be sent to the image display section 35, and generates the special light observation image on the basis of predetermined image processing.

The special light observation image is generated by using the blue region included in the first illuminating light P1, and the green region included in the second illuminating light P2 among the components included in the illuminating light P.

The calculator 34 sends the generated special light observation image to the image display section 35. As a result, the image display section 35 displays the special light observation image in, e.g., the CRT, the liquid crystal display, etc.

As described above according to the above first embodiment, in the normal light observation mode, when the second laser diode 6 is lit, the reflected light image of each frame which is obtained by the imaging of the imaging section 30 is stored in the second frame memory 33, and the stored reflected light image is calculated to generate the normal light observation image of the subject Q when the subject Q is irradiated with the white light. On the other hand, in the special light observation mode, the first and second laser diodes 5 and 6 are alternately lit to store the reflected light image of each frame which is obtained by the imaging of the imaging section 30 alternately in the first and second frame memories 32 and 33, the reflected light images stored in both of the first and second frame memories 32 and 33 are read out, and each reflected light image is calculated to generate the special light observation image.

Thus, when the normal light observation mode is input as the observation mode information M, it is possible to acquire the normal light observation image. When the special light observation mode is input as the observation mode, it is possible to acquire the special light observation image in which the blood vessel K is emphasized and displayed with a high contrast, by irradiating the subject Q with the two illuminating light P1 and P2.

The second illuminating light P2 has a component that becomes the white light by the blue laser light of the center wavelength of 445 nm (λ2) and the yellow fluorescence having the peak in the vicinity (λ3) of the wavelength of 565 nm. In these light, the yellow fluorescence includes the light in the vicinity of the wavelength of 540 nm. The light in the vicinity of the wavelength of 540 nm is scattered to a certain degree during the irradiation of the biological tissue, but travels deeper under the skin of the biological tissue than the blue-violet laser light with a wavelength of 415 nm (λ1) to be absorbed and scattered in the blood vessel K, etc. present under the skin, so that the light is effective for the observation of the blood vessel K of the subcutaneous tissue. Preferably, the peak wavelength λ3 of the component of the yellow fluorescence is present in the wavelength band of the absorption coefficient that is 1/2 or more of the absorption coefficient of the absorption peak wavelength of 540 nm of hemoglobin of the green region, which results in the increase of the ratio at which the light is absorbed in the observation of the blood vessel K of the subcutaneous tissue and the increase in the direction of the sensitivity of the G pixel, so that the high-contrast image can be obtained.

Specifically, the second illuminating light P2 is the white light including the blue laser light of the center wavelength of 445 nm ($\lambda$2) and the yellow fluorescence having the peak in the vicinity ($\lambda$3) of the wavelength of 565 nm. In the spectral component of the second illuminating light P2, there are included the blue laser light of the 420 nm wavelength in the vicinity of the absorption peak of hemoglobin and the absorption peak of 540 nm of hemoglobin of the green region, and thus, combination with the image acquiring device 3 can realize the special light observation. In particular, when the information of the B color of the special light observation image is acquired, the information is acquired in a state where the subject is irradiated only with the primary light in the first illuminating light P1 as the B color region, and therefore it is possible to obtain the special light observation image in which the blood vessel K of the surface of the biological tissue is shown in high contrast.

In the spectral component of the second illuminating light P2, the white light also including red light is achieved, and thus the combination with the predetermined image acquiring device 3 can realize the normal light observation.

The first illuminating light P1 emitted in the normal light observation mode and the second illuminating light P2 emitted in the special light observation mode are emitted from the same wavelength conversion unit 12, so downsizing can be achieved. The structure is especially effective for a nasal endoscope, etc., when there is a restriction on light sources such as the first and second laser diodes 5 and 6, or a region where the wavelength conversion unit 12 is disposed.

The image acquisition in the special light observation mode has a composition in which the images are acquired in two image frames, but the special light image may be composed of one of only the B pixel information of a first frame image stored in the first frame memory 32 and only the G pixel information of a second frame image stored in the second frame memory 33.

The second laser diode 6 is lit as the second illuminating light P2 to emit the blue-violet laser light as the primary light, but the first laser diode 5 may also be lit to emit the blue laser light as the primary light. In this case, a shape of a spectrum of the second illuminating light P2 changes, and thus it is possible to perform regulation of the color by regulating the intensity of the blue laser light emitted from the first laser diode 5.

There may be a structure in which the first illuminating light P1 and the second illuminating light P2 are always switched to irradiate the subject irrespective of the observation mode information M, and the reflected light image of each frame obtained by the imaging of the imaging section 30 is successively and continuously stored in the first and second frame memories 32 and 33. In this structure, in accordance with the observation mode information M, the calculator 34 may switch an operation of constructing the normal light observation image from the reflected light image stored in the second frame memory 33 or an operation of combining the respective reflected light images stored in the first frame memory 32 and the second frame memory 33 to construct the special light observation image. In addition, as a structure having two calculators 34 and two image display sections 35, there may be a structure in which the normal light observation image and the special light observation image are acquired and displayed in parallel.

In the present embodiment, hemoglobin is used as a target substance, but another substance that is present in the body may be used, or a fluorescent probe to be applied from the outside of the body may be used. In this case, an absorption wavelength region of the fluorescent probe may be matched with a wavelength of the excitation light. The fluorescent probe is applied from the outside of the subject and responds to a specific wavelength to emit light.

As blue laser emitted from a semiconductor laser, the laser having a peak wavelength in a wavelength region of 400 nm to 440 nm in which the absorption coefficient of hemoglobin is large may be used.

When the sensitivity peak of the blue region of the CCD pixels is present on the side of a wavelength longer than the wavelength of 440 nm, the peak wavelength of the blue laser light is more preferably present between wavelengths of 415 nm and 440 nm of the absorption peaks of hemoglobin, because the high-contrast bright image of the blood vessel K can easily be obtained.

The wavelength converting member 22 is not limited to the oxide fluorescent material (YAG, TAG) having the Ce (cerium) activated garnet crystal structure, and an Eu activated oxynitride fluorescent material, or an Eu activated sulfide fluorescent material or the like is usable as long as the fluorescent material absorbs the light of the blue band and emits broad yellow fluorescence.

When the light quantity in the blue region of 450 nm to 480 nm in the second illuminating light P2 is 1/5 or less of the light quantity in the wavelength region of 400 nm to 440 nm, less blue light components are received by the G pixels, and the components have less influence as the special light image noise. Preferably, a ratio of the light quantity is 1/10 or less.

[Second Embodiment]

Next, a second embodiment of the present invention will be described with reference to the drawings. The same parts as in FIG. 1 are denoted with the same reference signs and detailed descriptions thereof are omitted.

FIG. 9 shows a configuration diagram of the wavelength conversion unit 12 in the subject observation device including the light source device. The wavelength conversion unit 12 has a constitution of two color light, and includes two wavelength converting members 22-1 and 22-2. One wavelength converting member 22-1 absorbs blue laser light of the center wavelength of 445 nm ($\lambda$2) which is emitted as primary light from the second laser diode 6, to emit fluorescence of a green region.

The wavelength converting member 22-1 is made of, e.g., a Ce activated oxide fluorescent material. The wavelength converting member 22-1 is not excited by blue-violet laser light with the wavelength of 415 nm ($\lambda$1) which is emitted as the primary light from the first laser diode 5. FIG. 10 shows absorption/fluorescence characteristics of the wavelength converting member (a green color) 22-1.

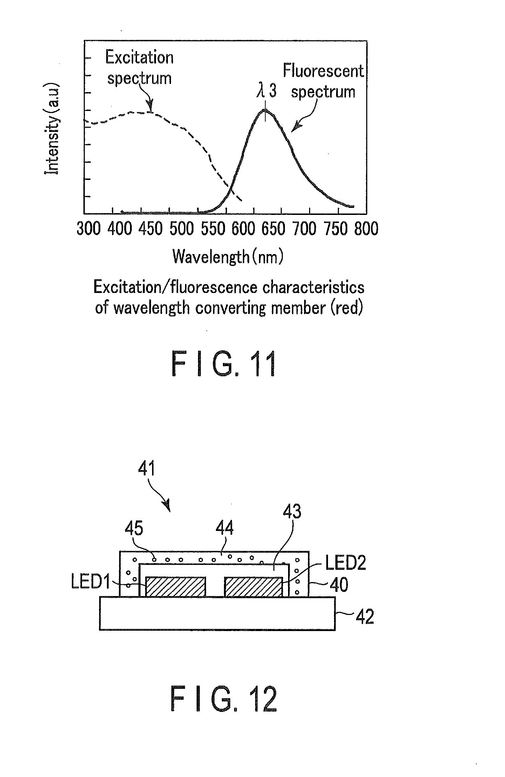

The other wavelength converting member 22-2 absorbs the blue-violet laser light with the wavelength of 415 nm ($\lambda$1) which is emitted from the first laser diode 5 or the blue laser light with the center wavelength of 445 nm ($\lambda$2) which is emitted from the second laser diode 6, to emit fluorescence of a red region. The wavelength converting member 22-2 is made of, e.g., an Eu activated nitride fluorescent material. FIG. 11 shows absorption/fluorescence characteristics of the wavelength converting member (a red color) 22-2.

The other wavelength converting member 22-2 as a fluorescent member may be made of a fluorescent material which does not absorb the blue-violet laser light of the wavelength of 415 nm ($\lambda$1) emitted from the first laser diode 5, but absorbs green fluorescence to emit the light. The wavelength converting member 22-2 may be made of a fluorescent material which absorbs the blue laser light with the center wavelength of 445 nm ($\lambda$2) which is emitted as the primary light from the second laser diode 6 to emit the light.

By use of the wavelength conversion unit 12, when the first laser diode 5 is lit and the blue-violet laser light with the wavelength of 415 nm ($\lambda$1) is emitted from the first laser diode 5, the one wavelength converting member 22-1 is not excited by the blue-violet laser light with the wavelength of 415 nm ($\lambda$1), but transmits the blue-violet laser light. In addition, the other wavelength converting member 22-2 absorbs the primary light of the blue-violet laser light with the wavelength of 415 nm ($\lambda$1) emitted from the first laser diode 5 to emit the fluorescence of the red region.

Due to the above, the wavelength conversion unit 12 emits first illuminating light P1 of a white color in which the blue-violet laser light transmitted through the one wavelength converting member 22-1 and the fluorescence of the red region emitted by the other wavelength converting member 22-2 are mixed at a predetermined ratio.

On the other hand, when the second laser diode 6 is lit and the blue laser light of the center wavelength of 445 nm ($\lambda$2) is emitted from the second laser diode 6, the one wavelength converting member 22-1 absorbs the primary light of the blue laser light of the center wavelength of 445 nm ($\lambda$2) emitted from the second laser diode 6 to emit the fluorescence of the green region. At this time, the one wavelength converting member 22-1 transmits a part of the blue laser light with the center wavelength of 445 nm ($\lambda$2) which does not contribute to the emission of the fluorescence of the green region. In addition, the other wavelength converting member 22-2 absorbs the primary light of the blue laser light with the center wavelength of 445 nm ($\lambda$2) emitted from the second laser diode 6 to emit the fluorescence of the red region.

As a result of the above, the wavelength conversion unit 12 emits second illuminating light P2 in which a part of the blue laser light transmitted through the one wavelength converting member 22-1, the fluorescence of the green region emitted by the wavelength converting member 22-1, and the fluorescence of the red region emitted by the other wavelength converting member 22-2 are mixed at a predetermined ratio.

According to such a structure, in a normal light observation mode, when the second laser diode 6 is lit, the wavelength conversion unit 12 emits the second illuminating light P2 of the white color in which a part of the blue laser light transmitted through the one wavelength converting member 22-1, the fluorescence of the green region emitted by the wavelength converting member 22-1, and the fluorescence of the red region emitted by the other wavelength converting member 22-2 are mixed at the predetermined ratio. A subject Q is irradiated with the second illuminating light P2.

At this time, a reflected light image of each frame, which is obtained by imaging of the imaging section 30, is stored in the second frame memory 33, and the stored reflected light image is calculated to generate a normal light observation image of the subject Q when the subject Q is irradiated with the white light.

On the other hand, in a special light observation mode, when the first and second laser diodes 5 and 6 are alternately lit and when the first laser diode 5 is lit, the wavelength conversion unit 12 emits the first illuminating light P1 in which the blue-violet laser light transmitted through the one wavelength converting member 22-1 and the fluorescence of the red region emitted by the other wavelength converting member 22-2 are mixed at the predetermined ratio.

When the second laser diode 6 is lit, the wavelength conversion unit 12 emits the second illuminating light P2 in which a part of the blue laser light transmitted through the one wavelength converting member 22-1, the fluorescence of the green region emitted by the wavelength converting member 22-1, and the fluorescence of the red region emitted by the other wavelength converting member 22-2 are mixed at the predetermined ratio.

Due to the above, the subject Q is successively irradiated alternately with the first illuminating light P1 and the second illuminating light P2. At this time, the reflected light image of each frame obtained by the imaging of the imaging section 30 is stored alternately in the first frame memory 32 and the second frame memory 33. The respective reflected light images stored in both of the first and second frame memories 32 and 33 are read out from the respective frame memories 32 and 33 and calculated by the image processing section 31 to generate a special light observation image.

As described above, according to the above second embodiment, the wavelength conversion unit 12 is formed of the one wavelength converting member 22-1 that absorbs the primary light of the blue laser light with the center wavelength of 445 nm (λ2) emitted from the second laser diode 6 to emit the fluorescence of the green region and is not excited by the primary light of the blue-violet laser light with the wavelength of 415 nm (λ1) emitted from the first laser diode 5, and the other wavelength converting member 22-2 that absorbs the blue-violet laser light with the wavelength of 415 nm (λ1) emitted from the first laser diode 5 or the primary light of the blue laser light with the center wavelength of 445 nm (λ2) emitted from the second laser diode 6 to emit the fluorescence of the red region.

Consequently, in addition to the above effect of the first embodiment, the second illuminating light P2 becomes the white light in which a part of the blue laser light transmitted through the one wavelength converting member 22-1, the fluorescence of the green region emitted by the wavelength converting member 22-1, and the fluorescence of the red region emitted by the other wavelength converting member 22-2 are mixed at the predetermined ratio, i.e., the white light having a flat spectrum from the green region to the red region, so that color rendering properties in the normal light observation mode improve.

In the present second embodiment, as compared with the above first embodiment, a spectral component of the red region is present up to a long wavelength side, thus enabling red tissues which are often present in a biological tissue of the subject Q to be observed with high reproducibility.

The second illuminating light P2 also includes the red fluorescence, but the special light observation image can be prepared without exerting any influence on a pixel signal output from a G pixel for use during image construction in the special light observation mode.

The wavelength conversion unit 12 is not limited to the structure in which the wavelength converting members 22-1 and 22-2 are laminated in this order to an entrance direction of the primary light of the blue laser light and the blue-violet laser light, and the wavelength converting members 22-2 and 22-1 may be laminated in this order.

[Third Embodiment]

Next, a third embodiment of the present invention will be described with reference to the drawings. The same parts as in FIG. 1 are denoted with the same reference signs and detailed descriptions thereof are omitted.

FIG. 12 shows a configuration diagram of a light source device 41. In the light source device 2, the first and second laser diodes 5 and 6 in the above respective embodiments are replaced with first and second light emitting diodes LED1 and LED2 and the above wavelength converting member 22 is replaced with a wavelength converting material 40 to integrate and constitute the light source device 31.

In the light source device 41, a substrate 42 is disposed. On the substrate 42, the first and second light emitting diodes LED1 and LED2 are disposed.

The first light emitting diode LED1 corresponds to the first laser diode 5. The first light emitting diode LED1 emits, for example, LED light (first excitation light) of a blue-violet color of primary light in a wavelength region of a first spectrum.

The second light emitting diode LED2 corresponds to the second laser diode 6. The second light emitting diode LED2 emits, for example, LED light (second excitation light) of a blue color of primary light in a wavelength region of a first spectrum.

On the substrate 42, the wavelength converting material 40 is disposed to cover the first and second light emitting diodes LED1 and LED2. A portion between the wavelength converting material 40 and each of the light emitting diodes LED1 and LED2 is filled with a first resin 43. The first resin 43 is formed of a light transmitting resin to guide, to the wavelength converting material 40, the respective LED light emitted from the first and second light emitting diodes LED1 and LED2, respectively.

The wavelength converting material 40 receives the respective LED light emitted from the respective light emitting diodes LED1 and LED2 to emit first or second illuminating light P1 or P2. The wavelength converting material 40 includes a second resin 44 and fluorescent materials 45 scattered in the second resin 44.

Each of the fluorescent materials 45 receives each LED light of the primary light emitted from the first and second light emitting diodes LED1 and LED2, i.e., the LED light of a blue-violet color, the LED light of a blue color, or mixed LED light in which the respective LED light are mixed, and emits the first or second illuminating light P1 or P2 as illuminating light P in accordance with each LED light.

Also in the light source device 41 in which the first and second light emitting diodes LED1 and LED2 are used, an effect similar to the above effect of the first embodiment can be produced.

It is to be noted that the present invention is not limited to the above embodiments as they are, and in an implementation stage, structural elements can be modified and embodied without departing from the gist of the present invention. Additionally, by appropriate combination of the structural elements disclosed in the above embodiments, various inventions can be formed. For example, several structural elements may be deleted from all the structural elements described in the embodiments. Furthermore, the structural elements of the different embodiments may appropriately be combined.

**Claims**

1. A subject observation system **characterized by** comprising:

   primary light sources;
   a wavelength converting section which converts wavelengths of plural primary light of different wavelengths emitted from the primary light sources, respectively;
   an image acquiring section which performs color separation by color regions to acquire an image of an irradiated region of illuminating light in an observation target, when the observation target is irradiated with the illuminating light including the light whose wavelength has been converted by the wavelength converting section;
   a mode input section which is able to input observation modes; and
   a light source section which successively lights the primary light sources to successively irradiate the observation target with the plural illuminating light from an aperture of a same position, when a special light observation mode to emphasize and display a specific observation target in the observation target is input into the mode input section,
   wherein the image acquiring section acquires the images every time the observation target is irradiated with respective illuminating light, and generates a special light observation image of the observation target based on the images.

2. The subject observation system according to claim 1, **characterized in that** when the special light observation mode is input into the mode input section, the image acquiring section acquires the special light observation image based on information of a narrowband spectrum element generated by overlap of wavelength characteristics of the plural illuminating light output from the light source section with wavelength characteristics of the color regions possessed by the image acquiring section.

3. The subject observation system according to claim 2, **characterized in that**
   the light source section emits first illuminating light and second illuminating light having different wavelengths, respectively, as the plural illuminating light, and
   when the special light observation mode is input into the mode input section,
   the image acquiring section acquires information of a first narrowband spectrum element from the image of each first imaging frame which is acquired every time the observation target is irradiated with the first illuminating light from the light source section,
acquires information of a second narrowband spectrum element from the image of each second imaging frame which is acquired every time the observation target is irradiated with the second illuminating light from the light source section, and
combines the information of the first narrowband spectrum element and the information of the second narrowband spectrum element to construct the special light observation image.

4. The subject observation system according to claim 3, **characterized in that**
   the image acquiring section includes:

   a first frame memory which stores the image of the first imaging frame;
   a second frame memory which stores the image of the second imaging frame; and
   an imaging section which images the observation target, and
   the image acquiring section stores, in the first frame memory, the image of the first imaging frame which is acquired by exposure processing in the imaging section when the observation subject is irradiated with the first illuminating light by the light source section, and stores, in the second frame memory, the image of the second imaging frame which is acquired by the exposure processing in the imaging section when the observation subject is irradiated with the second illuminating light by the light source section.

5. The subject observation system according to claim 3 or 4, **characterized in that** the first narrowband spectrum element and the second narrowband spectrum element include wavelengths each having a high absorption intensity of a substance of the observation target, and belonging to different absorption peaks, respectively.

6. The subject observation system according to claim 5, **characterized in that**
   the first illuminating light includes at least the first narrowband spectrum element constituted of first primary light emitted from a first primary light source in the primary light sources, and
   in a first color region in the color regions including the first narrowband spectrum element, the absorption intensity of the substance of the observation target is low at the wavelength that is relatively hard to be absorbed.

7. The subject observation system according to claim 5, **characterized in that**

the second illuminating light includes light in which second primary light emitted from a second primary light source in the primary light sources is mixed with first fluorescence generated by a first fluorescent material to be excited by the second primary light, the first fluorescent material is not excited by first primary light in the primary light sources, and the second primary light belongs to a first color region in the color regions including the first narrowband spectrum element, and has a wavelength different from that of the first primary light.

8.  The subject observation system according to claim 7, **characterized in that**
    a second color region in the color regions includes the second narrowband spectrum element,
    a short wavelength boundary value or a long wavelength boundary value of the second color region is present,
    the short wavelength boundary value or the long wavelength boundary value of the second color region is positioned between a long wavelength boundary value and a short wavelength boundary value of the first fluorescence,
    another boundary value of the second color region is positioned at a longer wavelength than the long wavelength boundary value or a shorter wavelength than the short wavelength boundary value of the first fluorescence, and
    the second narrowband spectrum element is a wavelength region where the second color region overlaps with a spectrum of the first fluorescence.

9.  The subject observation system according to claim 4, **characterized in that** the image acquiring section generates the image in the special light observation mode by use of pixel information of the first color region of the first imaging frame and pixel information of the second color region of the second imaging frame.

10.  The subject observation system according to claim 5 or 6, **characterized in that** the substance of the observation target includes hemoglobin that is present in the subject.

11.  The subject observation system according to claim 5 or 6, **characterized in that** the substance of the observation target includes a fluorescent probe applied from the outside of the subject to respond to a specific wavelength, thereby emitting the light.

12.  The subject observation system according to claim 6, **characterized in that** the first illuminating light has an intensity in a wavelength region that is included in a visible light wavelength region and not included in the first color region, in addition to the first primary light.

13.  The subject observation system according to claim 12, **characterized in that** the first illuminating light includes a second fluorescence to be excited by the first primary light, in addition to the first primary light.

14.  The subject observation system according to claim 7, **characterized in that** the second illuminating light has an intensity at a wavelength that is not included in the second color region.

15.  The subject observation system according to claim 14, **characterized in that** the second illuminating light includes a third fluorescence to be excited by the second primary light.

16.  The subject observation system according to claim 14 or 15, **characterized in that**
    the second illuminating light is white light, and
    the image obtained in the second imaging frame is a normal light observation image.

17.  The subject observation system according to claim 8, **characterized in that**
    the first primary light has an intensity peak at a wavelength in a vicinity of 415 nm,
    the second secondary light has an intensity peak at a wavelength in a vicinity of 445 nm,
    the first fluorescence is green fluorescence to be excited by the second primary light,
    the first color region is a blue region based on a wavelength of 460 nm, and
    the second color region is a green region based on a wavelength of 540 nm.

18.  The subject observation system according to claim 13, **characterized in that** the second fluorescence includes red fluorescence to be excited by the first primary light.

19.  The subject observation system according to claim 15, **characterized in that** the third fluorescence includes red fluorescence to be excited by the second primary light.

20.  The subject observation system according to claim 7, **characterized in that** the second illuminating light includes the first illuminating light.

21.  The subject observation system according to claim 1, **characterized in that** the primary light sources are included in the light source section, and include laser light sources, respectively.

22.  The subject observation system according to claim 1, **characterized in that** the primary light sources included in the light source section include light emitting diodes, respectively.

**23.** The subject observation system according to claim 4, **characterized in that** the image acquiring section performs calculation processing of luminance information of a specific detection color screen in the image of the first imaging frame stored in the first frame memory and luminance information of a specific detection color screen in the image of the second imaging frame stored in the second frame memory, to analytically obtain an image by a specific wavelength component, thereby generating the special light observation image.

**24.** The subject observation system according to claim 16, **characterized in that**

the mode input section is able to input a normal light observation mode to perform normal observation to the observation target, and

when the normal light observation mode is input into a mode instructing section, the light source section outputs the second illuminating light as the illuminating light.

**25.** A subject observation method **characterized by** comprising:

converting wavelengths of plural primary light of different wavelengths which are emitted from primary light sources, respectively;
irradiating an observation target with plural illuminating light including light whose wavelength has been converted;
performing color separation by color regions to acquire images of irradiated regions with the plural illuminating light in the observation target;
successively lighting the primary light sources to successively irradiate the observation target with the plural illuminating light from an aperture of a same position, in a special light observation mode to emphasize and display a specific observation target in the observation target;
acquiring the images every time the observation target is irradiated with respective illuminating light; and
generating a special light observation image of the observation target based on the images.

F I G. 1

F I G. 2

Excitation/fluorescence characteristics
of wavelength converting member

F I G. 3

Spectral sensitivity characteristics of imaging means

# FIG. 4

Spectral intensity characteristics P($\lambda$)
of second illuminating light P2

# FIG. 5

λ1(415nm)

First excitation light

First fluorescence
(yellow fluorescence)

λ3(565nm)

Intensity

1

0.05

400  460  520  580  640  700

Wavelength(nm)

Spectral intensity characteristics P(λ)
of first illuminating light P1

## FIG. 6

λh1

One example of
biological tissue

λh2

(1/5 region)

(1/2 region)

Hemoglobin

Absorption coefficient(cm⁻¹)

10000

1000

100

10

1

400  460  520  580  640  700

Wavelength(nm)

Absorption characteristics of biological tissue

## FIG. 7

F I G. 8

F I G. 9

Excitation/fluorescence characteristics
of wavelength converting member (green)

F I G. 10

Excitation/fluorescence characteristics
of wavelength converting member (red)

# F I G. 11

# F I G. 12

## INTERNATIONAL SEARCH REPORT

International application No.

PCT/JP2014/055235

A. CLASSIFICATION OF SUBJECT MATTER
*A61B1/00*(2006.01)i, *A61B1/04*(2006.01)i, *A61B1/06*(2006.01)i

According to International Patent Classification (IPC) or to both national classification and IPC

B. FIELDS SEARCHED

Minimum documentation searched (classification system followed by classification symbols)
A61B1/00, A61B1/04, A61B1/06

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2009-297141 A (Fujifilm Corp.), 24 December 2009 (24.12.2009), entire text; all drawings & US 2009/0306478 A1 & US 2010/0280322 A1 & EP 2130484 A1 & EP 2241244 A1 & DE 602009001103 D & AT 506000 T | 1-24 |
| Y | JP 2012-170774 A (Fujifilm Corp.), 10 September 2012 (10.09.2012), entire text; all drawings (Family: none) | 1-24 |
| Y | JP 2012-100887 A (Fujifilm Corp.), 31 May 2012 (31.05.2012), entire text; all drawings (Family: none) | 1-24 |

☒ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
|---|---|
| Date of the actual completion of the international search<br>13 March, 2014 (13.03.14) | Date of mailing of the international search report<br>25 March, 2014 (25.03.14) |
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2014/055235

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2013-22341 A  (Fujifilm Corp.), 04 February 2013 (04.02.2013), paragraphs [0031] to [0035], [0044] to [0063], [0075] to [0077], [0089], [0098] to [0101]; fig. 2 to 11 & US 2013/0030268 A1    & EP 2550910 A1 & CN 102894948 A | 1-24 |
| X | JP 2011-50470 A  (Fujifilm Corp.), 17 March 2011 (17.03.2011), paragraphs [0039], [0051] to [0057], [0070] to [0075], [0084] to [0085]; fig. 6, 13(B) (Family: none) | 1,21 |
| X | JP 2012-70839 A  (Fujifilm Corp.), 12 April 2012 (12.04.2012), paragraphs [0027] to [0038], [0044] to [0058], [0084] to [0095]; fig. 2 to 3 (Family: none) | 1,21 |
| A | JP 2009-153712 A  (Olympus Corp.), 16 July 2009 (16.07.2009), paragraphs [0011] to [0017]; all drawings & US 2009/0167149 A1    & EP 2074934 A2 | 17 |

Form PCT/ISA/210 (continuation of second sheet) (July 2009)

**INTERNATIONAL SEARCH REPORT**

| International application No. |
| --- |
| PCT/JP2014/055235 |

**Box No. II        Observations where certain claims were found unsearchable (Continuation of item 2 of first sheet)**

This international search report has not been established in respect of certain claims under Article 17(2)(a) for the following reasons:

1. ☒ Claims Nos.:  25
   because they relate to subject matter not required to be searched by this Authority, namely:
   (See extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2014/055235

Continuation of Box No.II-1 of continuation of first sheet(2)

The invention relating to a method for observing a subject as set forth in claim 25, said method comprising a step for, while using a device (a device for observing a subject) in the human body, radiating illumination light to an observation object and acquiring an image to observe the subject, pertains to methods for treatment of the human body by surgery as well as diagnostic methods.  Thus, claim 25 relates to a subject matter which this International Searching Authority is not required, under the provisions of Article 17(2)(a)(i) of the PCT and Rule 39.1(iv) of the Regulations under the PCT, to search.

Form PCT/ISA/210 (extra sheet) (July 2009)

**EP 2 965 683 A1**

**Patent documents cited in the description**

- JP 2005205195 A